# EUROPEAN PATENT APPLICATION

(11) **EP 1 785 110 A1**
(43) Date of publication of application: **16.05.2007**
(21) Application number: 06123255.9
(22) Date of filing: 31.10.2006
(51) Int. Cl.: A61F 9/007, A61B 17/02, A61B 17/34

(54) **Sclerotomy adapter**

(30) Priority: 09.11.2005 US 270398
(71) Applicant: Alcon, Inc., 6331 Hünenberg (CH)
(72) Inventor: Charles, Steven T., Memphis, TN 38119 (US); Hickingbotham, Dyson W., Stouchsburg, PA 19567 (US)
(74) Representative: Hanna, Peter William Derek

(57) **Abstract**

The invention provides a thin-walled, hubbed cannula (14) for use as an adapter (10) suitable for placement in an incision of the sclera, having having an outer diameter that is approximately the same as a 20 gauge surgical instrument and an inner diameter defining a bore (16) that is slightly larger than a 23 gauge surgical instrument. The scleral contact surface (20) of the hub (12) of the adapter also can be rounded to provide a pivot point for a surgical instrument shaft inserted in the bore (16), thereby reducing bending forces on the relatively thin shaft of the instrument.

## Description

### Background of the Invention

This invention relates generally to the field of microsurgery and, more particularly, to posterior segment ophthalmic microsurgery.

Current vitreoretinal techniques in which surgical instruments are inserted into the eye require the dissection of the conjunctiva and the creation of pars plana scleral incisions through the sclera. The dissection of the conjunctiva typically involves pulling back the conjunctiva about the eye so as to expose large areas of the sclera. Following the creation of the incisions, surgical instruments are passed through these incisions and the inserted instruments are observed through the pupil using a microscope and corrective optics. These instruments are used to manipulate and/or dissect retinal tissues within the eye as well as to implement the specific retinal treatment technique (e.g., photocoagulation). Many scleral incisions created for vitreoretinal surgery are made large enough to accommodate the required instruments, the inserted portions being typically 19 or 20 gauge (approximately 1 mm) in diameter. After completing the specific treatment procedure, the inserted instruments are removed from the incisions in the sclera. Because the incisions through the sclera are large enough to pass 19 or 20 gauge instruments, the incisions are typically too large to self-seal. Thus, the incisions must be sutured shut. Following the suturing of the scleral incisions, the surgical personnel reposition the conjunctiva in its normal position and suture the conjunctival incision. While such methods and techniques have proven to be effective in the treatment of vitreoretinal disease, there is a strong motivation to move away from procedures requiring sutures and instead look to greatly simplified sutureless procedures. Therefore, recently some surgical instruments have been miniaturized so that the cannulas or shafts of the instruments are on the order of 23 or 25 gauge. While many surgeons prefer to use smaller gauge instruments, larger gauge instruments must sometime s also be used during a surgical procedure. This requires that the sclerotomy be made larger enough to accommodate the larger instruments. The larger incision; however, may leak when smaller gauge instruments are used later in the procedure.

Therefore, a need continues to exist for an adapter for a sclerotomy that facilitates the use of smaller gauge instrument with incisions sized to accommodate larger gauge instruments.

### Brief Summary of the Invention

The present invention improves upon the prior art by providing a thin walled, hubbed cannula with an exterior diameter of approximately that of a 20 gauge surgical instrument and in interior bore having a diameter that is slightly larger than a 23 gauge or 25 gauge surgical instrument. The scleral contact surface of the hub of the adapter also can be rounded to provide a pivot point for the surgical instrument shaft, thereby reducing bending forces on the relatively thin shaft of the instrument.

Accordingly, one objective of the present invention is to provide a sclerotomy adapter.

Another objective of the present invention is to provide a thin walled cannula with a hub.

Another objective of the present invention is to provide a sclerotomy adapter that helps reduce bending forces on a surgical instrument.

These and other advantages and objectives of the present invention will become apparent from the detailed description and claims that follow.

### Brief Description of the Drawing

FIG. 1 is an end view of the sclerotomy adapter of the present invention.
FIG. 2 is a longitudinal cross-sectional view of the sclerotomy adapter of the present invention.

### Detailed Description of the Preferred Embodiments

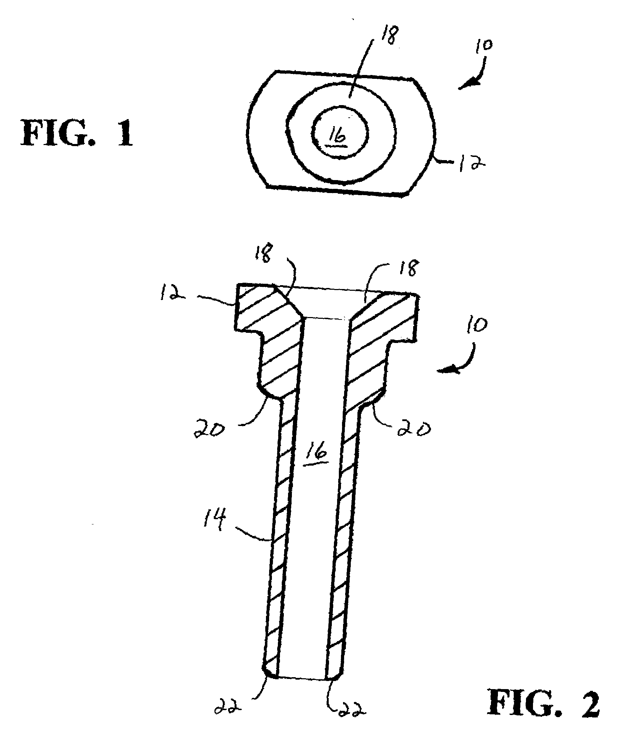

As best seen in FIGS. 1 and 2, sclerotomy adapter 10 of the present invention generally includes hub 12 integrally formed with cannula or shaft 14. Adapter 10 may be formed of any suitable material such as stainless steel or titanium, but molded or machined thermoplastic is preferred. Adapter 10 contains bore 16 that extends the entire length of adapter 10 through hub 12 and cannula 14. Hub 12 may be round or oval in plan view with a minor diameter of approximately between 0.50 inches and 0.70 inches and a major diameter of approximately between 0.80 inches and 0.10 inches. Cannula 14 extends approximately between 0.100 inches and 0.200 inches distally from hub 12, with between approximately 0.145 inches being most preferred. Preferably, tip 22 of cannula 14 is rounded for ease of insertion into an incision. Cannula 14 has an outside diameter that approximates the outer diameter of 20 gauge surgical instruments, on the order of between 0.034 inches and 0.038 inches. Bore 16 has a diameter that is slightly larger than the outer diameter of 23 gauge or 25 gauge surgical instruments. Hub 12 contains chamfered or beveled portion 18 at the entrance to bore 16, for example, cut at a 45° angle, which assists in the insertion of a surgical tool into bore 16. Hub 12 also contains rounded shoulders 20 that contact the incision and prevent hub 12 from entering the incision. Shoulders 20 also help provide a pivot point for adapter 10 that assists in preventing bending of a surgical tool while being used with adapter 10.

This description is given for purposes of illustration and explanation. It will be apparent to those skilled in the relevant art that changes and modifications may be made to the invention described above without departing from its scope or spirit.

## Claims

1. A sclerotomy adapter (10), comprising:
a) a hub (12); and
b) a cannula (14) connected to the hub, the cannula having an outer diameter that is approximately the same as a 20 gauge surgical instrument and an inner diameter defining a bore (16) that is slightly larger than a 23 gauge surgical instrument.

2. The adapter of claim 1 wherein the hub (12) defines a rounded shoulder (20) portion, adapted in use to contact an incision, prevent the hub from entering the incision, and help to provide a pivot point for the adapter that assists in preventing bending of a surgical instrument inserted in the said bore (16) while being used with the adapter.

3. The adapter of claim 1 or claim 2, wherein the cannula (14) defines a rounded tip (22).

4. The adapter of any of claims 1 to 3, wherein the hub (12) defines a chamfered or bevelled portion (18) at the entrance to the inner diameter bore (16).

5. The adapter of any of claims 1 to 3, wherein the outer diameter of the cannula (14) is between 0.864 mm (0.034 inches) and 0.965 mm (0.038 inches).
